(19) Europäisches Patentamt
European Patent Office
Office européen des brevets

(11) Publication number: **0 003 619**
**B1**

(12) **EUROPEAN PATENT SPECIFICATION**

(45) Date of publication of patent specification: 05.05.82

(21) Application number: 79200001.0

(22) Date of filing: 02.01.79

(51) Int. Cl.$^3$: C 07 D 271/10, C 07 D 263/22, C 07 D 233/70, C 07 D 231/04, A 01 N 43/74, A 01 N 43/82, A 01 N 43/48

# (54) Heterocyclic-substituted anilide derivatives, a process for their preparation, herbicidal compositions containing them and a method of controlling undesired plant growth using them.

(30) Priority: 09.01.78 US 867748

(43) Date of publication of application: 22.08.79 Bulletin 79/17

(45) Publication of the grant of the patent: 05.05.82 Bulletin 82/18

(84) Designated Contracting States: BE CH DE FR GB IT NL SE

(56) References cited:
FR - A - 1 588 718
FR - A - 2 225 426
FR - A - 2 334 674
GB - A - 1 446 084

Chemie der Pflanzenschutz und Schädlings-Bekämpfungsmittel, Vol. 5 (1977), Wegler and Ene, pages 247—251.

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Pilgram, Kurt Hans Gerhard
2607 Warwick Lane
Modesto California 95350 (US)

(74) Representative: Rogers, Roy Charles et al,
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 003 619 B1

Heterocyclic-substituted anilide derivatives, a process for their preparation, herbicidal compositions containing them and a method of controlling undesired plant growth using them

This invention relates to heterocyclic-substituted anilide derivatives, a process for their preparation, herbicidal compositions containing them and a method of controlling undesired plant growth using them.

A variety of anilide derivatives are known to have herbicidal activity. For example, "Chemie der Pflanzenschutzund Schädlings-Bekämpfungsmittel" Vol. 5 (1977) (Wegler and Eue) at pages 247—251 describes a number of herbicidal anilide derivatives including several which are available commercially. Anilide derivatives having different biological properties are disclosed in French Patent Specification 1,588,718 where it is taught that certain cyclopropane-carbonilides are useful as agents to combat insects, arachnids, nematodes, fungi, bacteria and mollusks.

The present invention provides a compound of the general formula

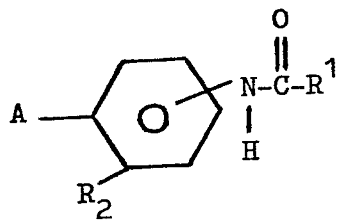

I

wherein

$R^1$ represents a hydrogen atom, a cyclopropyl group having a hydrogen fluorine, chlorine or bromine atom or a methyl or a methoxy group in its 1-position, or an alkyl group having 1 to 4 carbon atoms or an alkenyl group having 2 or 3 carbon atoms which groups may be unsubstituted or substituted by one or more fluorine, chlorine and/or bromine atoms; $R^2$ represents a fluorine, chlorine or bromine atom, a trifluoromethyl, nitro or cyano group or an alkyl, alkoxy or alkylthio group having from 1 to 6 carbon atoms; and A represents one of the groups:

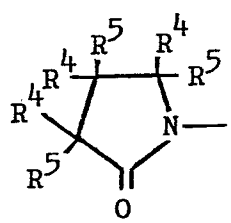
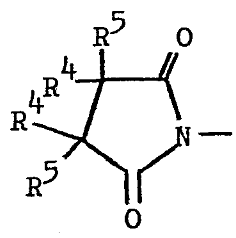
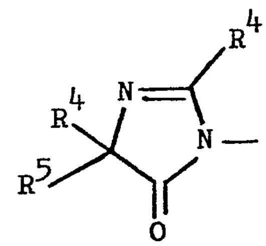
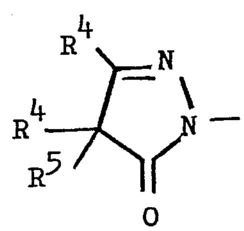
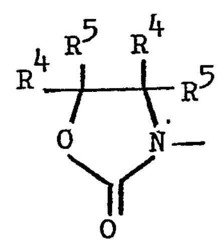
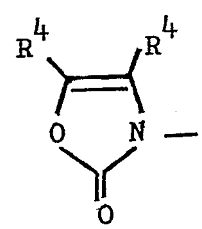
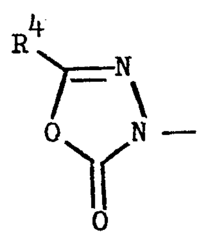
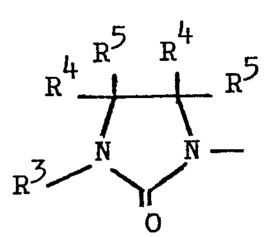
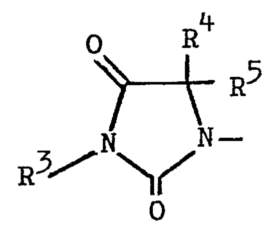
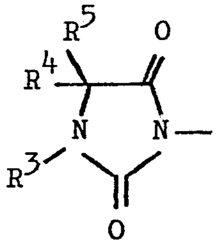
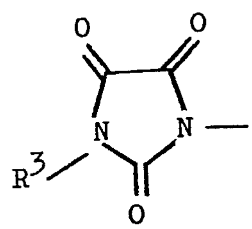
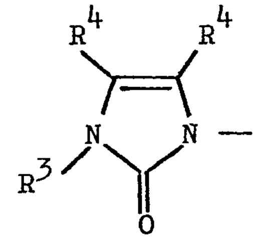

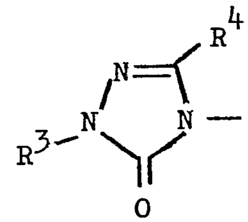

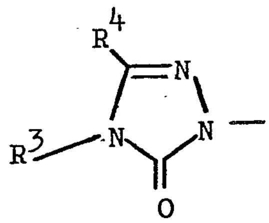

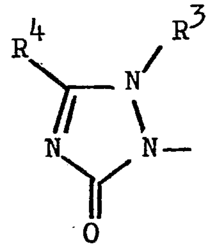

or 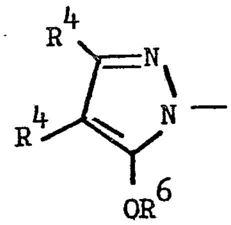

in which $R^3$ represents a hydrogen atom or an alkyl group having from 1 to 6 carbon atoms; each $R^4$ independently represents a hydrogen atom, a cyclopropyl or 1-methyl cyclopropyl group, or an alkyl group having from 1 to 6 carbon atoms which may be unsubstituted or substituted by one or more halogen atoms, or, if two $R^4$ groups are present bonded to adjacent carbon atoms, the two $R^4$ groups together with the interjacent carbon atoms represent a hydrocarbyl ring having 5 or 6 carbon atoms; each $R^5$ independently represents a hydrogen atom or an alkyl group having 1 or 2 carbon atoms; and $R^6$ represents an alkyl or acyl group having from 1 to 6 carbon atoms; or a salt thereof.

Preferably, $R^1$ represents for example a methyl or ethyl group, or a 2-propen-2-yl group. Compounds wherein $R^1$ represents an unsubstituted cyclopropyl group, or, preferably, a 1-methylcyclopropyl group, are especially preferred.

Preferably the group

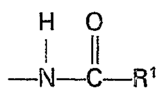

is located in a position meta- or, especially, para-, relative to the heterocyclic group A in the compounds of the general formula I.

Preferably, $R^2$ represents a chlorine or bromine atom or a methyl or trifluoromethyl group.

Preferably $R^3$ represents a hydrogen atom or an alkyl group having 1 to 4 carbon atoms; preferably $R^4$ represents a hydrogen atom, an alkyl group having 1 to 4 carbon atoms, or a cyclopropyl or 1-methylcyclopropyl group, or, if two $R^4$ groups are present bonded to adjacent carbon atoms, the two $R^4$ groups together with the interjacent carbon atoms form a hydrocarbyl ring having 5 or 6 carbon atoms, which may be saturated or unsaturated; preferably $R^5$ represents a hydrogen atom or a methyl group; and preferably $R^6$ represents a cyclopropylcarbonyl or a 1-methylcyclopropylcarbonyl group. Thus, examples of preferred compounds of the general formula I include those wherein $R^3$ represents a hydrogen atom or a methyl group; $R^4$ represents a hydrogen atom or a tertiary-butyl, methyl, cyclopropyl or 1-methylcyclopropyl group, and $R^5$ represents a hydrogen atom or a methyl group.

Further examples of preferred compounds according to the invention are those compounds wherein $R^1$ represents a hydrogen atom or a methyl, ethyl, tertiarybutyl, 2-propen-2-yl, cyclopropyl or 1-methylcyclopropyl group; $R^2$ represents a chlorine or bromine atom or a trifluoromethyl group; and A represents a group of formula

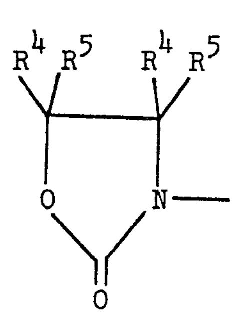

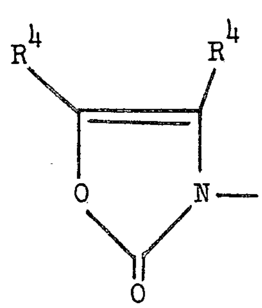

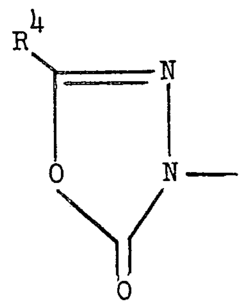

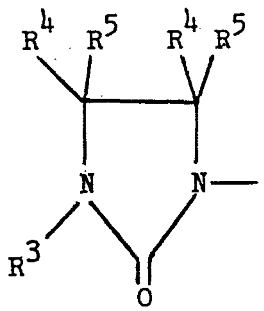 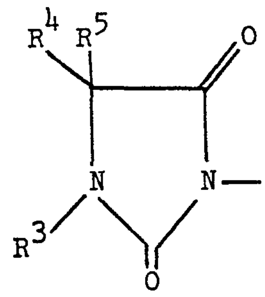 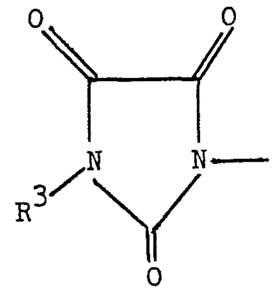

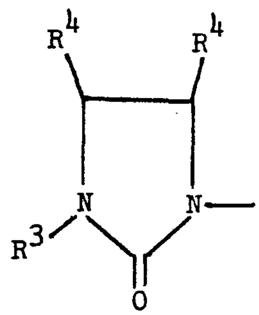

or

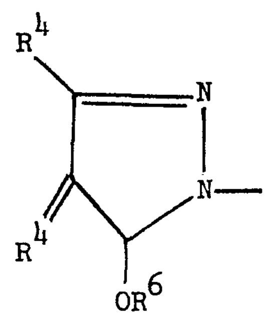

wherein

$R^3$ represents a hydrogen atom or a methyl group, each $R^4$ independently represents a hydrogen atom or a methyl, trifluoromethyl, tertiary-butyl, 2,2-dimethylpropyl or 1-methylcyclopropyl group, or, if two $R^4$ groups are present bonded to adjacent carbon atoms, the two $R^4$ groups together with the interjacent carbon atoms represent a benzene ring; each $R^5$ independently represents a hydrogen atom or a methyl group; and $R^6$ represents a 1-methylcyclopropylcarbonyl group.

The invention also provides a process for the preparation of a compound of the general formula I, characterised in that one hydrogen atom of the amino group in a compound of the general formula

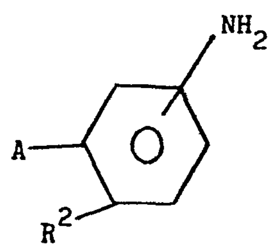

II

wherein

A and $R^2$ have the meanings given for the general formula I is replaced with a group of the general formula

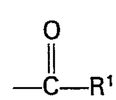

wherein

$R^1$ has the meaning given for the general formula I, by reaction with a suitable acylating agent.

Suitable acylating agents include acyl chlorides of the general formula $R^1$.CO.Cl. Suitably the compound of the general formula II is reacted with the acyl chloride in the presence of an acid binding agent, for example in the presence of approximately one mole of ethylamine.

Free acids of the general formula $R^1CO_2H$ and the anhydrides thereof are also suitable acylating agents. For example, compounds of the general formula I wherein $R^1$ represents a hydrogen atom may be prepared by reacting the compound of the general formula II with formic acid, preferably formic acid containing not more than 10% by weight of water, at elevated temperature.

Salts of compounds of the general formula I may be prepared by any suitable method, for example by reaction of a free base of the general formula I with an acid, for example a mineral acid.

The compounds of the general formula II can be prepared by reducing the nitro group in a compound of the general formula

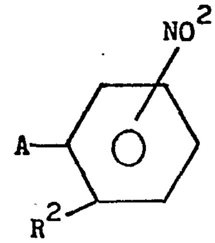

III

wherein

A and $R^2$ have the meanings given for the general formula I. Any suitable reducing agent may be used, for example hydrogen in the presence of a catalyst, for example Raney-nickel or palladium charcoal. Acetic acid and iron powder is also a convenient reducing agent, the reduction in this case suitably being carried out in refluxing water containing a small amount of acetic acid.

The compounds of the general formula III may be prepared by processes known in the art or by analogous processes.

The compounds of the invention have been found to be useful for controlling undesired plant growth. Certain compounds of the general formula I have been found to be herbicidally effective against a wide range of plant species while others have been found to be effective only against a limited number of plant species and are therefore of interest as selective herbicides. Some of the compounds exhibit a high degree of herbicidal activity in the control of a variety of economically important species of grasses and broadleaved weeds, for example, control of grasses and broadleaf weeds in soybean and cotton crops can be achieved by post-emergence application of such compounds of the invention as N-(3-bromo-4-(5-tert-butyl)-2-oxo-1,3,4-oxadiazol-3(2H)-yl)phenyl)-1-methylcyclopropanecarboxamide, while other compounds of the invention have shown post-emergence and, in some cases, pre-emergence selective herbicidal activity in crops such as peanuts, grain sorghum, cotton, rice, corn and alfalfa.

The invention therefore also provides a herbicidal composition comprising at least one carrier and, as active ingredient, at least one compound according to the invention. The invention further provides a method of controlling undesired plant growth at a locus which comprises applying to the locus a herbicidally effective amount of a compound according to the invention or of a herbicidal composition according to the invention.

A carrier is a solid or liquid material, which may be inorganic or organic and of synthetic or natural origin, with which the active compound is mixed or formulated to facilitate its application to the plant, seed, soil or other object to be treated, or its storage, transport or handling.

Suitable solid carriers are natural and synthetic clays and silicates, for example natural silicas such as diatomaceous earths; magnesium silicates, for example talcs and magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Examples of suitable liquid carriers are water; alcohols, for example isopropanol and glycols; ketones, for example, acetone, methyl ethyl ketone, methyl isobutyl ketone and cyclohexanone; ethers; aromatic hydrocarbons, for example benzene, toluene and xylene; petroleum fractions, for example kerosene and light mineral oils; chlorinated hydrocarbons, for example carbon tetrachloride, perchloroethylene and trichloroethane. Compounds which are normally gaseous but which have been compressed to form a liquid may be used. Mixtures of different liquids are often suitable.

The carrier may be a surface-active agent, or if more than one carrier is present, at least one of the carriers may be a surface-active agent. The surface-active agent may be an emulsifying agent, a dispersing agent or a wetting agent; it may be nonionic or ionic. Any of the surface-active agents usually applied in formulating herbicides or insecticides may be used. Examples of suitable surface-active agents are the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxides.

The compositions of the invention may for example be formulated as wettable powders, dusts, granules, solutions, emulsifiable concentrates, emulsions, suspension concentrates and aerosols. Wettable powders are usually compounded to contain 25, 50 or 75% by weight of active ingredient and usually contain in addition to solid inert carrier, 3—10% by weight of a dispersing agent, 15% of a surface-active agent and where necessary, 0—10% by weight of stabiliser(s) and/or other additives such as penetrants or stickers. Dusts are usually formulated as a dust concentrate having a similar composition to that of a wettable powder but without a dispersant or surface-active agent, and are diluted in the field with further solid carried to give a composition usually containing 1/2—10% by weight of active ingredient. Granules are usually prepared to have a size between 10 and 100 BS mesh (1.676—0.152 mm), and may be manufactured by agglomeration or impregnation techniques. Generally granules will contain 1/2—25% by weight active ingredient and 0—10% by weight of additives such as stabilisers, slow release modifiers and binding agents. Emulsifiable concentrates usually contain, in addition to the solvent and, when necessary, co-colvent, 10—50% weight per volume active ingredient, 2—20% weight per volume emulsifiers and 0—20% weight per volume of appropriate additives such as stabilisers, penetrants and corrosion inhibitors. Suspension concentrates are compounded so as to obtain a stable, non-sedimenting, flowable product and usually contain 10—75% w active ingredient, 0.5—5% w of dispersing agents, 1—5% w of surface-active agent, 0.1—10% w of suspending agents such as protective colloids and thixotropic agents, 0—10% w of appropriate additives such as defoamers, corrosion inhibitors, stabilisers, penetrants and stickers, and water or an organic liquid in which the active ingredient is substantially insoluble; certain organic solids or inorganic salts may be dissolved in the carrier to assist in preventing sedimentation or as anti-freeze agents for water.

Aqueous dispersions and emulsions, for example, compositions obtained by diluting a wettable powder or a concentrate according to the invention with water, also lie within the scope of the present invention. The said emulsions may be of the water-in-oil or the oil-in-water type, and may have a thick mayonaise-like consistency.

The compositions of the invention may also contain other ingredients, for example other compounds possessing insecticidal, acaricidal, herbicidal or fungicidal properties.

The amount of compound of the invention to be used in controlling the undesired vegetation will naturally depend on the condition of the vegetation, the degree of activity desired, the formulation used, the mode of application, the climate, the season of the year, and other variables. Recommendations as to precise amounts are, therefore, not possible. In general, however, application to the locus to be protected of from approximately 0.1 to 10.0 Kg per hectare of the compound according to the invention will be satisfactory.

The following Examples illustrate the invention. In these Examples, the identities of compounds, intermediates and final, were confirmed by elemental analysis, and infrared and nuclear magnetic spectral analyses as necessary.

## Example 1

N - (3 - chloro - 4 - (2 - (1 - methylcyclopropyl) - 5 - oxo - $\Delta^2$ - 1,3,4 - oxadiazolin - yl)phenyl) - 1 - methylcyclopropanecarboxamide

a) 5-(1-Methylcyclopropyl)-1,3,4-oxadiazol-2(3H)-one.

A solution containing 54.0 g (0.385 mol) of 1-methylcyclopropanecarboxylic acid hydrazide (m.p. 72—74°C) and 90 g (0.9 mol) of phosgene in 1000 ml of ethyl acetate was refluxed for 2 hours. The reaction mixture was concentrated on a rotary evaporator. The residual solid crystallized from ether-hexane (1:5) to give 56.0 g (85%) of product as a white crystalline solid; m.p. 77—78°C.

b) 3-(2-chloro-4-nitrophenyl)-5-(1-methylcyclopropyl)-1,3,4-oxadiazol-2(3H)-one.

A mixture containing 16.3 g (0.116 mol) of 1a) and 22.4 g (0.116 mol) of 1,2-dichloro-4-nitrobenzene in 250 ml of dimethylformamide was stirred during the portionwise addition of 4.9 g (0.116 mol) of 57% sodium hydride-oil paste. After 0.5 hour, the mixture was heated at 130° for 1.5 hours, cooled and poured into ice water. The aqueous layer was acidified with hydrochloric acid and extracted with ether. Concentration of the dried ether extract followed by purification using silica chromatography gave 21.5 g (62.5%) of product as a light yellow crystalline solid; m.p. 95—97°C (from ether).

c) 3-(4-amino-2-chlorophenyl)-5-(1-methyl-cyclopropyl)-1,3,4-oxadiazol-2(3H)-one.

A mixture containing 19 g (0.064 mol) of 1b) above and 50 ml of glacial acetic acid in 950 ml of water was stirred and refluxed during portionwise addition of 20 g of iron powder. The reaction mixture was filtered while hot, and the filtrate was cooled and extracted with ether. The dried ether extract was concentrated to dryness. Crystallization of the residue from ether-hexane gave 5.5 g (32%) of product as a white crystalline solid; m.p. 195—196°C.

d) N - (3 - chloro - 4 - (2 - (1 - methylcyclopropyl) - 5 - oxo - $\Delta^2$ - 1,3,4 - oxadiazolin - ) - yl)phenyl) - 1 - methylcyclopropanecarboxamide.

2.9 of 1c) above was treated with 1.0 g of 1-methylcyclopropanecarbonyl chloride in 50 ml of ether containing 1.0 g of triethylamine to give 1.4 g (54%) of product as a white solid; m.p. 130—131°C (from ether).

Example 2

N - (4 - (2 - oxo - 3 - oxazolidinyl) - 3 - (trifluoromethyl)phenyl) - 1 - methylcyclopropanecarboxamide

a) 3-(4-Nitro-3-(trifluoromethyl)phenyl)-2-oxazolidinone

To a stirred solution containing 8.7 g (0.1 mol) of 2-oxazolidinone and 22.6 g (0.1 mol) of 2-chloro-5-nitrobenzotrifluoride in 200 ml of dimethylformamide was added portionwise, at 20—25°C, 4.2 g (0.1 mol) of 57% sodium hydride in oil. This addition was exothermic to 45°C. After 2 hours at ambient temperature, the reaction mixture was heated at 100°C for 2 hours, poured into ice water, neutralized with hydrochloric acid and extracted with ether. The ether was dried and evaporated. The residual oil was crystallized from ether to give 3.5 g (14%) of product as a light tan solid; m.p. 112—113°C.

b) 3-(4-amino-3-(trifluoromethyl)phenyl)-2-oxazolidinone.

An ethanolic solution of 3.1 g (11 mmol) of 2a) above was reduced over a palladium-charcoal catalyst in a Parr shaker to give 2.5 g (90%) of product as a white solid; m.p. 170—171°C.

c) N-(4-(2-oxo-3-oxazolidinyl)-3-(trifluoromethyl)phenyl)-1-methylcyclopropanecarboxamide.

A mixture containing 2.0 g (8 mmol) of 2b) above, 2.5 g of triethylamine and 2.0 g of 1-methylcyclopropanecarbonyl chloride in 50 ml of tetrahydrofuran was refluxed for 2 hours. The reaction mixture was poured into water and extracted with ether, and the extract was dried and concentrated. Recrystallization of the residue from ether gave 2.0 g (75%) of product as a white crystalline solid; m.p. 146—147°C.

Example 3

N-(3-bromo-4-(2-(tert-butyl)-5-oxo-$\Delta^2$-1,3,4-oxadiazolin-yl)phenyl)acetamide

a) Pivalic acid 2-(2-bromo-4-nitrophenyl)-hydrazide.

To a stirred and chilled (0°) solution containing 200 g (0.86 mol) of 2-bromo-4-nitrophenylhydrazine (m.p. 143—145°) and 116.8 g (0.9 mol) of ethyldiisopropylamine in 1000 ml of tetrahydrofuran was added dropwise 108.5 g (0.9 mol) of pivaloyl chloride. The reaction mixture was stirred at ambient temperature for 1 hour and then concentrated to dryness under reduced pressure. The residue was treated with water, filtered and recrystallized from ethanol to give 165 g (52%) of product as a light yellow solid; m.p. 145°C.

b) 3-(2-Bromo-4-nitrophenyl)-5-(tert-butyl)-1,3,4-oxadiazol-2(3H)-one.

A solution containing 160 g (0.506 mol) of 3a) above and 198 g (2.0 mol) of phosgene in 900 ml of ethyl acetate was refluxed (60°) for 120 hours. The reaction mixture was concentrated. The residue was triturated with hexane to give 97.3 g (57%) of product as a light yellow crystalline solid; m.p. 99.5°C.

c) 3-(2-Bromo-4-aminophenyl)-5-tert-butyl)-1,3,4-oxadiazol-2(4H)-one

A mixture containing 34.2 g (0.1 mol) of 3b) above and 150 ml of glacial acetic acid in 1500 ml of water was stirred and refluxed during the portion-wise addition of 80 g of iron powder. After 15 minutes, the reaction mixture was cooled (25°) and filtered. The filter cake was extracted with warm (60°) ethanol. The ethanolic extract was concentrated to about 150 ml and diluted with water to give 29.8 g (9%) of product as a white solid; m.p. 170—172°C.

d) N-(3-bromo-4-(2-(tert-butyl)-5-oxo$\Delta^2$-1,3,4-oxadiazolin-yl)-phenyl)acetamide.

To a solution of 4.05 g (0.013 mol) of 3c) above and 1.82 g (0.018 mol) of triethylamine in 75 ml of ether was added with stirring 1.26 g (0.016 mol) of acetyl chloride. After 15 minutes, the reaction mixture was diluted with water and acidified by the dropwise addition of hydrochloric acid. The ethereal layer was separated, dried, and concentrated. Recrystallization of the residue from ether-hexane gave 3.78 g (82%) of product as a tan solid; m.p. 144—145°C.

Example 4

N-(3-chloro-4-(2-(tert-butyl)-5-oxo-$\Delta^2$-1,3,4-oxadiazolin-4-yl)phenyl)formamide

a) Pivalic acid 2-(2-chloro-4-nitrophenyl)hydrazide.

To a stirred and chilled (0°C) solution containing 25 g (0.133 mol) of 2-chloro-4-nitrophenylhydrazine and 18.06 g (0.14 mol) of ethyldiisopropylamine in 200 ml of tetrahydrofuran was added dropwise 16.75 g (0.139 mol) of pivaloyl chloride. The reaction mixture was briefly heated to reflux and then concentrated under reduced pressure. The residue was treated with water, filtered and dried to give 32.5 g (90% yield) of the desired product as a yellowish solid; m.p. 123°C.

b) 3-(2-Chloro-4-nitrophenyl)-5-(tert-butyl)-1,3,4-oxadiazol-2(3H)-one.

A solution containing 94 g (0.346 mol) of 4a) above and 99 g (1.0 mol) of phosgene in 1000 ml of ethyl acetate was refluxed for about 80 hours. The reaction mixture was evaporated to dryness. Recrystallization of the residue from ether-hexane gave 102.9 g (53% yield) of the desired product as a yellow solid; m.p. 98°C.

c) 3-(2-Chloro-4-aminophenyl)-5-(tert-butyl)-1,3,4-oxadiazol-2(3H)-one.

A mixture containing 54.6 g (0.184 mol) of 4b) above and 110 ml of glacial acetic acid in 1540 ml of water was stirred and refluxed during the portionwise addition of 25 g of iron powder. After about 3/4 of an hour, the reaction mixture was cooled and filtered. The filter cake was extracted with ethanol at 50—60°C. The ethanol extracts were concentrated and the residue was triturated with water to give

42.4 g (86% yield of yellow product; m.p. 158—159°C.

d) N-(3-chloro-4-(2-(tert-butyl)-5-oxo-$\Delta^2$-1,3,4-oxadiazolin-4-yl)phenyl)formamide.

A solution containing 10 g (0.037 mol) of 4c) above in 100 ml of 98—100% formic acid was refluxed for 1/2 hour, poured over ice water and extracted with ether. The ethereal extracts were washed well with water, dried, filtered and concentrated. The residual oil was triturated with hexane, filtered and dried to give 9.13 g (84% yield) of the desired product as a white solid; m.p. 124.5—125.5°C.

Example 5

N-(4-(3-methyl-2-oxo-1-imidazolinyl)-3-(trifluoromethyl)phenyl)-1-methylcyclopropanecarboxamide

a) 1-(2-Hydroxyethyl)-1-methyl-3-(4-nitro-3-(trifluoromethyl)phenyl) urea.

To a solution of 46.4 g (0.2 mol) of 4-nitro-3-(trifluoromethyl)phenyl isocyanate in 200 ml of tetrahydrofuran was added dropwise with stirring and external cooling 15.2 g (0.2 mol) of 2-(methylamino)-ethanol. This addition was exothermic. The solution was concentrated under reduced pressure, washed with water, acidified with hydrochloric acid and extracted with ether. Crystallization of the ether extract gave 43.69 g (71% yield) of the desired product as a tan solid; m.p. 180—181°C.

b) 1-(2-Chloroethyl)-1-methyl-3-(4-nitro-2-(trifluoromethyl)phenyl) urea.

A solution containing 20 g (0.065 mol) of 5a) above and 10 g of thionyl chloride in 200 ml of benzene was refluxed for 1.5 hours. The reaction mixture was concentrated under reduced pressure. Recrystallization from ether-hexane gave 16 g (76% yield) of the desired product as a light yellow solid; m.p. 86—87°C.

c) 1-Methyl-3-(4-nitro-2-(trifluoromethyl)phenyl)-2-imidazolidinone.

To a suspension of 14.5 g (0.045 mol) of 5b) above in 100 ml of methanol was added a solution of 4 g of potassium hydroxide in 10 ml of water. The coloured mixture was refluxed for about 1/2 hour, cooled and filtered. The filtrate was concentrated to dryness, treated with water, acidified with hydrochloric acid and filtered to give 10 g (78% yield) of the desired product as a light yellow solid; m.p. 119—120°C.

d) 1-Methyl-3-(4-amino-2-(trifluoromethyl)phenyl)-2-imidazolidinone.

An ethanolic solution of 8.9 g (0.031 mol) of 5c) above was reduced in a Parr shaker over palladium-charcoal catalyst for about 3.5 hours at 50°C to give 7.54 g (94% yield) of desired product as an off-white solid; m.p. 132—133°C.

e) N - (4 - (3 - Methyl - 2 - oxo - 1 - imidazolidinyl) - 3 - (trifluoromethyl)phenyl) - 1 - methylcyclo - propanecarboxamide.

Reaction of 3.25 g (0.0125 mol) of 5d) above with 1.52 g (0.0128 mol) of 1-methylcyclopropanecarbonyl chloride in the presence of 1.31 g (0.013 mol) of triethylamine gave 3.53 g (83% yield) of desired product as a white solid; m.p. 90—91°C.

Example 6

N - (4 - (3 - Methyl - 2 - oxo - 4 - imidazolin - 1 - yl) - 3 - (trifluoromethyl)phenyl)acetamide

1 - (2,2 - Diethoxyethyl) - 1 - methyl - 3 - (4 - nitro - 2 - (trifluoromethyl)phenyl) urea.

To a solution of 46.4 g (0.2 mol) of 4-nitro-2- (trifluoromethyl)phenyl isocyanate in 100 ml of tetrahydrofuran was added 29 g (0.2 mol) of methylaminoacetaldehyde diethyl acetal. Recrystallization of the reaction product from hexane gave 67.2 g (89% yield) of the desired product as a light tan solid; m.p. 107—108°C.

b) 1-Methyl-3-(4-nitro-2-(trifluoromethyl)phenyl)-4-imidazolin-2-one.

A solution containing 63 g (0.167 mol) of 6a) above and 80 ml of 2-N-hydrochloric acid in 400 ml of ethanol was refluxed for 1.5 hours. The reaction mixture was concentrated under reduced pressure. Recrystallization from ether-hexane of the residue gave 41.7 g (81% yield) of the desired product as a light brown solid; m.p. 150—152°C.

c) 1-Methyl-3-(4-amino-2-(trifluoromethyl)phenyl)-4-imidazolin-2-one.

The reduction of 12.12 g (0.042 mol) of 6b) above in ethanol over palladium-charcoal catalyst at room temperature proceeded rapidly in a Parr shaker to give 3.0 g (28% yield) of the desired product as a white solid; m.p. 132—133°C.

d) N-(4-(3-methyl-2-oxo-4-imidazolin-1-yl)-3-(trifluoromethyl)phenyl)acetamide.

Treatment of a solution containing 10.46 g (0.04 mol) of triethylamine in 75 ml of tetrahydrofuran with 3.53 g (0.045 mol) of acetyl chloride proceeded smoothly to give 3.1 g (48% yield) of the desired product as a white solid; m.p. 192—194°C.

Example 7

N - (3 - Chloro - 4 - (3 - (trifluoromethyl) - 5 - (1 - methylcyclopropanecarbonyloxy) - 1 - pyrazolyl) - phenyl) - 1 - methylcyclopropanecarboxamide

a) Ethyl trifluoroacetoacetate 2-chloro-4-nitrophenyl hydrazone.

A mixture containing 40 g (0.213 mol) of 2-chloro-4-nitrophenylhydrazone and 39.2 g (0.213 mol) of ethyl trifluoroacetoacetate in 1600 ml of 50% aqueous ethanol and 50 ml of concentrated hydrochloric acid was stirred for about 12 hours at ambient temperature. The oil that had phase

separated was purified by silica chromatography to give 35.6 g (97% yield) of the desired product as a tan solid; m.p. 85.5—86.5°C.

b) 2-(2-Chloro-4-nitrophenyl)-2,4-dihydro-5-(trifluoromethyl)-3H-pyrazole-3-one.

A solution containing 29 g (0.082 mol) of 7a) above in 300 ml of ethanol containing 30 g of anhydrous hydrogen chloride was refluxed for about 8 days. The reaction mixture was concentrated under reduced pressure. Purification by silica chromatography of the residual solid gave 9.45 g (38% yield) of the desired product as an orange solid; m.p. 230—231°C.

c) 2-(4-Amino-2-chlorophenyl)-2,4-dihydro-5-(trifluoromethyl)-3H-pyrazole-3-one.

An ethanolic solution of 4.0 g (0.013 mol) of 7b) above was reduced over palladium-charcoal catalyst in a Parr shaker at 80°C to give 2.38 g (66% yield) of desired product as a tan solid; m.p. 275°C.

d) N - (3 - chloro - 4(3 - (trifluoromethyl) - 5 - (1 - methylcyclopropanecarbonyloxy) - 1 - pyrazolyl) - phenyl) - 1 - methylcyclopropanecarboxamide.

A mixture containing 4.05 g (0.0146 mol) of 7c) above, 1.7 g (0.017 mol) of triethylamine and 1.9 g (0.016 mol) of 1-methylcyclopropanecarbonyl chloride in 50 ml of ether was stirred at ambient temperature for about one hour. The mixture was concentrated and purified by silica chromatography to give 1.36 g (26% yield) of the desired product as a white solid; m.p. 128.5—129.5°C.

Example 8

N-(3-chloro-4-(4,4-dimethyl-2,5-dioxo-1-imidazolinyl)phenyl)cyclopropanecarboxamide

a) 1-(2-Chloro-4-nitrophenyl)-5-imino-4,4-dimethyl-2-imidazolidinone.

To a solution of 19.85 g (0.1 mol) of 2-chloro-4-nitrophenyl isocyanate in 150 ml of tetrahydrofuran was added dropwise 9.64 g (0.11 mol) of 2-amino-2-methylpropionitrile causing the internal temperature to rise to about 58°C. The reaction mixture was concentrated to dryness. Methanol (25 ml) and ether (150 ml) was added, causing the residual oil to crystallize. Filtration gave 27 g (96% yield) of the desired product as a white solid; m.p. 183—184°C.

b) 3-(2-Chloro-4-nitrophenyl)-5,5-dimethyl-2,4-imidazolidinedione.

A solution containing 25 g (0.0885 mol) of 8a) above in 500 ml of ethanol and 150 ml of 10% hydrochloric acid was refluxed for about 15 minutes and concentrated under reduced pressure to a volume of 100 ml. The mixture was cooled to about 10°C, filtered, washed with water and dried to give 21.25 g (75% yield) of the desired product as a white solid; m.p. 193—195°C.

c) 3-(4-Amino-2-chlorophenyl)-5,5-dimethyl-2,4-imidazolidinedione.

A mixture of 9.5 g (0.0335 mol) of 8b) above and palladium-charcoal catalyst was hydrogenated in a Parr shaker for about 2 hours at about 45°C to give 8.08 g (95% yield) of the desired product as a white solid; m.p. 249—250°C.

d) N-(3-chloro-4-(4,4-dimethyl-2,5-dioxo-1-imidazolidinyl)phenyl)cyclopropanecarboxamide.

To a solution of 2.0 g (0.00785 mol) of 8c) above in 250 ml of tetrahydrofuran was added 0.86 g (0.0082 mol) of cyclopropanecarbonyl chloride and 0.86 g (0.0085 mol) of triethylamine. This addition was exothermic to 40°C. The reaction mixture was diluted with water and extracted with ether. The organic layer was dried and concentrated to dryness. The residual solid was triturated with ether-hexane to give 1.94 g (76% yield) of desired product as a white solid; m.p. 259—260°C.

Example 9

N-(3-chloro-4-(3-methyl-2,4,5-trioxo-1-imidazolidinyl)phenyl)cyclopropanecarboxamide

a) 1-(2-Chloro-4-nitrophenyl)-3-methyl urea.

Anhydrous methylamine, 3.41 g (0.11 mol) was introduced through a gas-inlet tube into a stirred solution containing 19.85 g (0.1 mol) of 2-chloro-4-nitrophenyl isocyanate in 100 ml of tetrahydrofuran. This addition was exothermic. The reaction mixture was diluted with 500 ml of hexane, filtered and dried to give 22.5 g (99% yield) of desired product as a light yellow solid; m.p. 215—216°C.

b) 1-(2-Chloro-4-nitrophenyl)-3-methyl-2,4,5-imidazolidinetrione.

A mixture containing 21 g (0.091 mol) of 9a) above and 13 g (0.102 mol) of oxalyl chloride in 300 ml of toluene was refluxed for about 4 hours. The clear reaction mixture was concentrated under reduced pressure and triturated with methanol to give 25 g (97% yield) of desired product as a white solid; m.p. 133—134°C.

c) 1-(4-Amino-2-chlorophenyl)-3-methyl-2,4,5-imidazolidinetrione.

A mixture containing 2.5 g (0.0093 mol) of 9b) above and 0.5 g of 10% palladioum on charcoal in 200 ml of ethanol was hydrogenated in a Parr shaker for about 1.5 hours at 65°C and 40 pounds of hydrogen pressure to give 1.15 g (52% yield) of desired product as an off-white solid; m.p. 162—163°C.

d) N-(3-chloro-4-(3-methyl-2,4,5-trioxo-1-imidazolidinyl)phenyl)cyclopropanecarboxamide.

To a chilled (0°C) solution containing 2.23 g (0.0093 mol) of 9c) above in 25 ml of pyridine was added dropwise 1.1 g (0.0105 mol) of cyclopropanecarbonyl chloride causing the temperature to increase to 37°C. After about 10 minutes, the reaction mixture was diluted with ice water, and extracted with ether. The ether extracts were dried and concentrated. Trituration with ether-hexane

gave 1.0 g (35% yield) of desired product as a white solid; m.p. 212—213°C.

Examples 10—30

Using procedures similar to those of Examples 1 through 9, additional anilide derivatives were prepared as shown in Table 1 below:

TABLE I

ANILIDE DERIVATIVES

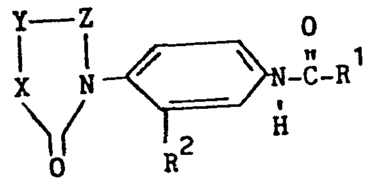

| Example | $R^1$ | $R^2$ | –X–Y–Z– | % Yield | M.p., °C |
|---|---|---|---|---|---|
| 10 | H | $CF_3$ | $C(CH_3)_3$<br>$-O-C=N-$ | 69 | 129–130 |
| 11 | $CH_3$ | Cl | $C(CH_3)_3$<br>$-O-C=N-$ | 42 | 154–155 |
| 12 | $CH_3$ | $CF_3$ | $C(CH_3)_3$<br>$-O-C=N-$ | 73 | 110–111 |
| 13 | $-C_2H_5$ | $CF_3$ | $C(CH_3)_3$<br>$-O-C=N-$ | 63 | 174–175 |
| 14 | $-C_2H_5$ | Cl | $C(CH_3)_3$<br>$-O-C=N-$ | 84 | 161–162 |
| 15 | $-C(CH_3)=CH_2$ | Cl | $C(CH_3)_3$<br>$-O-C=N-$ | 62 | 145–146 |
| 16 | $-C(CH_3)=CH_2$ | $CF_3$ | $C(CH_3)_3$<br>$-O-C=N-$ | 45 | 169–170 |
| 17 | 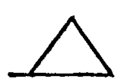 | Cl | $C(CH_3)_3$<br>$-O-C=N-$ | 69 | 166–167 |
| 18 | 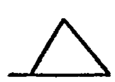 | Br | $CH_2C(CH_3)_3$<br>$-O-C=N-$ | 51 | 160–161 |
| 19 | 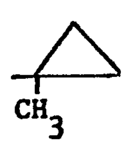 | $CF_3$ | $C(CH_3)_3$<br>$-O-C=N-$ | 76 | 157–158 |

TABLE I (Continued)

| Example | $R^1$ | $R^2$ | –X–Y–Z– | % Yield | M.p., °C |
|---|---|---|---|---|---|
| 20 | 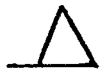 | Cl | $CH_3$<br>\|<br>–O–C=N– | 72 | 150–153 |
| 21 | 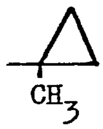 | Br | $CH_2C(CH_3)_3$<br>\|<br>–O–C=N– | 57 | 147–148 |
| 22 | 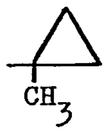 | Br | $C(CH_3)_3$<br>\|<br>–O–C=N– | 86 | 134–135 |
| 23 | 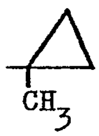 | Cl | $C(CH_3)_3$<br>\|<br>–O–C=N– | 51 | 132–133 |
| 24 | 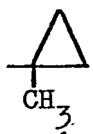 | Br | $-O-CH_2-CH_2-$ | 4 | 138–139 |
| 25 | 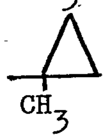 | Cl | $-O-CH_2CH_2-$ | 3 | 147–148 |
| 26 | 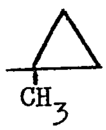 | $CF_3$ | 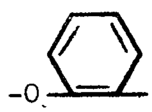 | 24 | 201–202 |
| 27 | 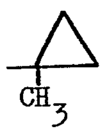 | $CF_3$ | $-N(CH_3)CH_2CH_2-$ | 32 | 180–181 |
| 28 | 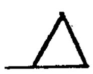 | $CF_3$ | $-N(CH_3)CH_2CH_2-$ | 24 | 120–121 |
| 29 | $C(CH_3)_3$ | Cl | $C(CH_3)_3$<br>\|<br>–O–C=N– | 75 | 155–156 |
| 30 | $C(CH_3)_3$ | | $C(CH_3)_3$<br>\|<br>–O–C=N– | 63 | 177–178 |

TABLE I (Continued)

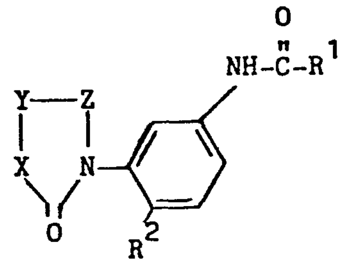

| Example | $R^1$ | $R^2$ | –X–Y–Z– | % Yield | M.p., °C |
|---|---|---|---|---|---|
| 31 | $CH_3$ | Cl | 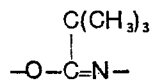 | 21 | 159–160 |
| 32 | $CH_3$ | Cl | 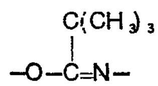 | 62 | 158–159 |

Example 33

Demonstration of Herbicidal Activity

The pre-emergence herbicidal activity of the compounds of the invention was evaluated by planting seeds of watergrass, garden cress, downey brome, velvet leaf, yellow foxtail, and sicklepod in test tubes, nominally measuring 25 x 200 millimetres, containing soil treated with the test compound at the rates of 0.1 and 1 mg per tube designated in Table II as Rates I and II, respectively. The planted soil was held under controlled conditions of temperature, moisture and light for 11 to 12 days. The amount of germination and growth in each tube were evaluated on a 0 to 9 scale, 0 rating indicating no effect, 9 death of the seedlings or no germination.

The post-emergence activity of the compounds of this invention was evaluated by spraying 7-day old crabgrass plants, 10-day old pigweed plants, 6-day old downey brome plants, 9-day old velvet leaf, 10-day old yellow foxtail plants and 7-day old sicklepod plants to runoff with a liquid formulation of the test compound at the rates of 0.8 millilitre of an 0.025% solution designated Rate I in Table II, and 0.8 millilitre of an 0.25% solution designated Rate II in Table II. The sprayed plants were held under controlled conditions for 10 to 11 days and the effect of the test compound was then evaluated visually, the results being rated on the 0 to 9 scale described above.

The results of the pre- and post-emergence tests are summarized in Table II.

TABLE II

HERBICIDE SCREEN RESULTS

| Compound of Example No. | Pre-emergence (Soil) | | | | | | | | | | | | Post-emergence (Foliar) | | | | | | | | | | | |
|---|---|---|---|---|---|---|---|---|---|---|---|---|---|---|---|---|---|---|---|---|---|---|---|---|
| | Watergrass | | Garden Cress | | Downey Brome | | Velvet Leaf | | Yellow Foxtail | | Sicklepod | | Crabgrass | | Pigweed | | Downey Brome | | Velvet Leaf | | Yellow Foxtail | | Sicklepod | |
| | I | II | I | II | I | II | I | II | I | II | I | II | I | II | I | II | I | II | I | II | I | II | I | II |
| 20 | 6 | 6 | 9 | 9 | 8 | 8 | 9 | 9 | 0 | 4 | 4 | 9 | 8 | 9 | 9 | 9 | 4 | 6 | 9 | 9 | 6 | 9 | 9 | 9 |
| 1 | 5 | 6 | 9 | 9 | 5 | 6 | 9 | 9 | 0 | 2 | 4 | 8 | 8 | 9 | 9 | 9 | 4 | 8 | 9 | 9 | 7 | 9 | 9 | 9 |
| 2 | 3 | 6 | 9 | 9 | 7 | 8 | 9 | 9 | 6 | 6 | 9 | 9 | 2 | 8 | 9 | 9 | 3 | 5 | 5 | 9 | 4 | 9 | 9 | 9 |
| 19 | 0 | 0 | 7 | 7 | 5 | 5 | 8 | 9 | 0 | 0 | 0 | 0 | 7 | 7 | 9 | 9 | 7 | 7 | 9 | 9 | 9 | 9 | 6 | 6 |
| 22 | 6 | 8 | 8 | 8 | 8 | 8 | 9 | 9 | 5 | 6 | 7 | 8 | 8 | 9 | 9 | 9 | 8 | 8 | 9 | 9 | 9 | 9 | 9 | 9 |
| 21 | 0 | 0 | 6 | 7 | 7 | 7 | 7 | 7 | 0 | 0 | 0 | 0 | 8 | 9 | 9 | 9 | 7 | 9 | 9 | 9 | 9 | 9 | 9 | 9 |
| 18 | 0 | 0 | 7 | 7 | 3 | 3 | 8 | 8 | 0 | 0 | 0 | 0 | 9 | 9 | 9 | 9 | 9 | 9 | 7 | 9 | 8 | 9 | 9 | 9 |

TABLE II (Continued)

| Compound of Example No. | Pre-emergence (Soil) | | | | | | | | | | | | Post-emergence (Foliar) | | | | | | | | | | | |
|---|---|---|---|---|---|---|---|---|---|---|---|---|---|---|---|---|---|---|---|---|---|---|---|---|
| | Watergrass | | Garden Cress | | Downey Brome | | Velvet Leaf | | Yellow Foxtail | | Sicklepod | | Crabgrass | | Pigweed | | Downey Brome | | Velvet Leaf | | Yellow Foxtail | | Sicklepod | |
| | I | II | I | II | I | II | I | II | I | II | I | II | I | II | I | II | I | II | I | II | I | II | I | II |
| 23 | 4 | 5 | 7 | 8 | 8 | 8 | 8 | 9 | 3 | 4 | 9 | 9 | 9 | 9 | 9 | 9 | 9 | 9 | 9 | 9 | 9 | 9 | 9 | 9 |
| 26 | 0 | 2 | 0 | 7 | 0 | 2 | 2 | 8 | 0 | 2 | 0 | 0 | 7 | 7 | 9 | 9 | 2 | 4 | 6 | 8 | 6 | 8 | 7 | 7 |
| 7 | 6 | 6 | 7 | 8 | 9 | 9 | 9 | 9 | 2 | 3 | 8 | 8 | 9 | 9 | 9 | 9 | 9 | 9 | 9 | 9 | 9 | 9 | 9 | 9 |
| 31 | 2 | 2 | 6 | 7 | 6 | 7 | 7 | 9 | 4 | 5 | 2 | 3 | 6 | 8 | 8 | 9 | 2 | 3 | 7 | 7 | 8 | 8 | 9 | 9 |
| 24 | 2 | 5 | 5 | 6 | 8 | 9 | 7 | 8 | 3 | 5 | 7 | 9 | 2 | 8 | 3 | 9 | 4 | 8 | 3 | 9 | 4 | 9 | 8 | 9 |
| 3 | 5 | 7 | 7 | 8 | 9 | 9 | 9 | 9 | 6 | 7 | 8 | 9 | 9 | 9 | 9 | 9 | 9 | 9 | 9 | 9 | 9 | 9 | 9 | 9 |
| 11 | 4 | 5 | 7 | 7 | 3 | 6 | 6 | 7 | 3 | 3 | 0 | 3 | 4 | 4 | 5 | 9 | 0 | 8 | 6 | 8 | 2 | 7 | 4 | 9 |

TABLE II (Continued)

| Compound of Example No. | Pre-emergence (Soil) | | | | | | | | | | | | Post-emergence (Foliar) | | | | | | | | | | | |
|---|---|---|---|---|---|---|---|---|---|---|---|---|---|---|---|---|---|---|---|---|---|---|---|---|
| | Watergrass | | Garden Cress | | Downey Brome | | Velvet Leaf | | Yellow Foxtail | | Sicklepod | | Crabgrass | | Pigweed | | Downey Brome | | Velvet Leaf | | Yellow Foxtail | | Sicklepod | |
| | I | II | I | II | I | II | I | II | I | II | I | II | I | II | I | II | I | II | I | II | I | II | I | II |
| 12 | 4 | 5 | 7 | 8 | 7 | 7 | 7 | 7 | 2 | 3 | 6 | 7 | 7 | 9 | 9 | 9 | 9 | 9 | 8 | 9 | 6 | 9 | 9 | 9 |
| 32 | 2 | 2 | 3 | 9 | 0 | 2 | 1 | 4 | 0 | 0 | 0 | 0 | 3 | 3 | 3 | 4 | 0 | 0 | 2 | 4 | 0 | 1 | 2 | 5 |

**Claims**

1. A compound of the general formula

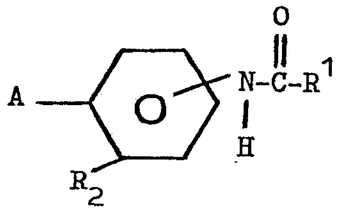

I

wherein

$R^1$ represents a hydrogen atom, a cyclopropyl group having a hydrogen, fluorine, chlorine or bromine atom or a methyl or methoxy group in its -1-position, or an alkyl group having 1 to 4 carbon atoms or an alkenyl group having 2 or 3 carbon atoms which groups may be unsubstituted or substituted by one or more, fluorine, chlorine and/or bromine atoms; $R^2$ represents a fluorine, chlorine or bromine atom, a trifluoromethyl, nitro or cyano group, or an alkyl, alkoxy or alkylthio group having from 1 to 6 carbon atoms; and A represents one of the groups:

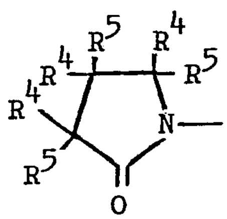
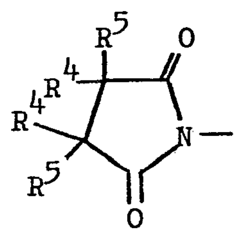
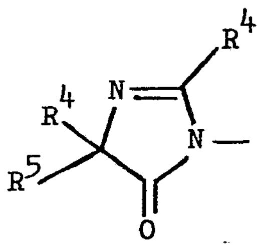
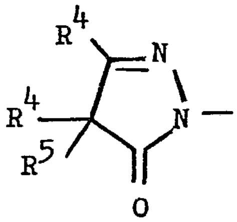
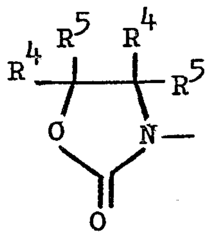
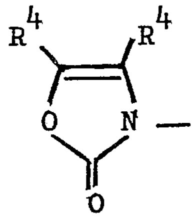
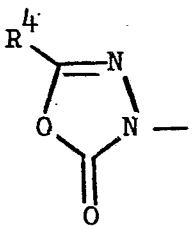
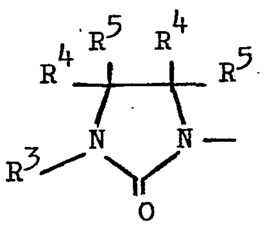
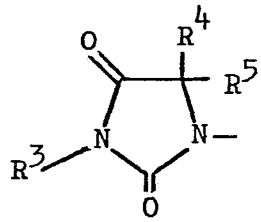
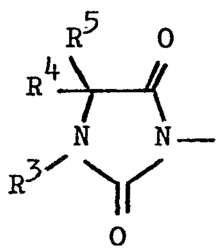
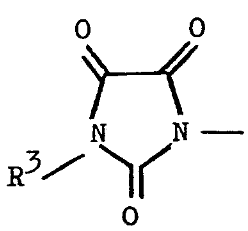
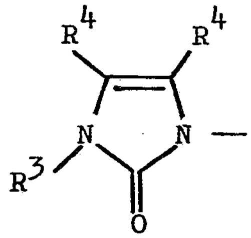
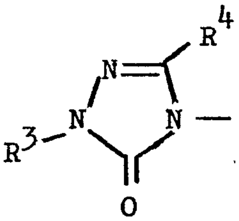
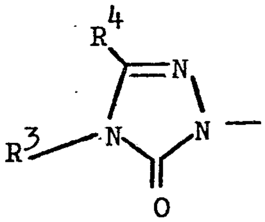
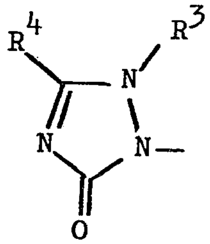

or

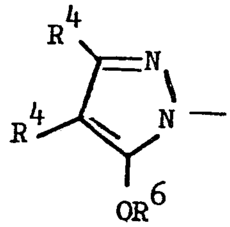

in which $R^3$ represents a hydrogen atom or an alkyl group having from 1 to 6 carbon atoms; each $R^4$ independently represents a hydrogen atom, a cyclopropyl or 1-methylcyclopropyl group, or an alkyl group having from 1 to 6 carbon atoms which may be unsubstituted or substituted by one or more halogen atoms, or, if two $R^4$ groups are present bonded to adjacent carbon atoms, the two $R^4$ groups together with the interjacent carbon atoms represent a hydrocarbyl ring having 5 or 6 carbon atoms; each $R^5$ independently represents a hydrogen atom or an alkyl group having 1 to 2 carbon atoms; and $R^6$ represents an alkyl or acyl group having from 1 to 6 carbon atoms; or a salt thereof.

2. A compound as claimed in claim 1, characterised in that $R^1$ represents a hydrogen atom, an alkyl group having 1 to 4 carbon atoms, an alkenyl group having 2 or 3 carbon atoms, or a cyclopropyl group having a hydrogen, fluorine, chlorine or bromine atom or a methyl or methoxy group in its 1-position.

3. A compound as claimed in claim 2, characterised in that the group

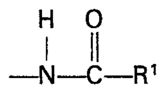

is located in a position meta- or para- relative to the group A.

4. A compound as claimed in any one of claims 1 to 3, characterised in that $R^2$ represents a chlorine or bromine atom or a methyl or trifluoromethyl group.

5. A compound as claimed in any one of claims 1 to 4, characterised in that $R^3$ represents a hydrogen atom or an alkyl group having 1 to 4 carbon atoms; each $R^4$ independently represents a hydrogen atom, an alkyl group having 1 to 4 carbon atoms, or a cyclopropyl or 1-methylcyclopropyl group, or if two $R^4$ groups are present bonded to adjacent carbon atoms, the two $R^4$ groups together with the interjacent carbon atoms form a hydrocarbyl ring having 5 or 6 carbon atoms; $R^5$ represents a hydrogen atom or a methyl group; and $R^6$ represents a cyclopropylcarbonyl or a 1-methylcyclopropylcarbonyl group.

6. A compound as claimed in claim 1, characterised in that $R^1$ represents a hydrogen atom or a methyl, ethyl, tertiary-butyl, 2-propen-2-yl, cyclopropyl or 1-methylcyclopropyl group; $R^2$ represents a chlorine or bromine atom or a trifluoromethyl group and A represents a group of formula

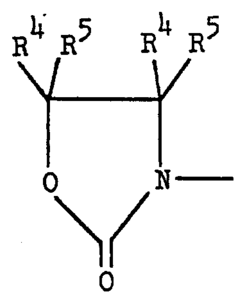

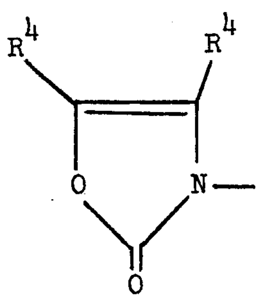

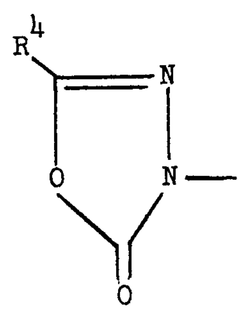

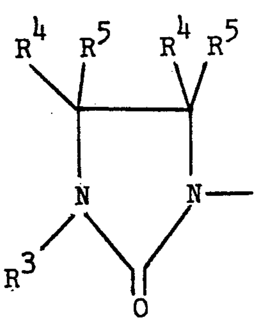

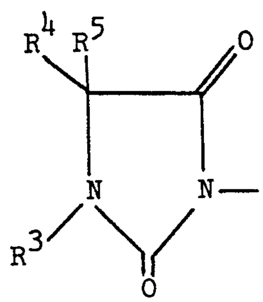

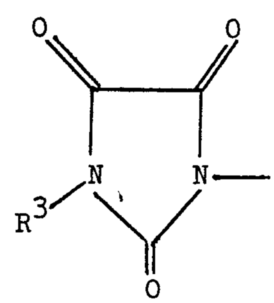

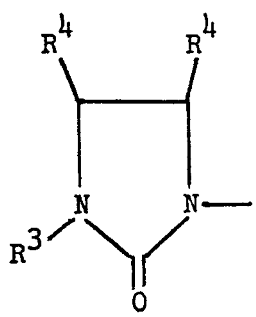

or

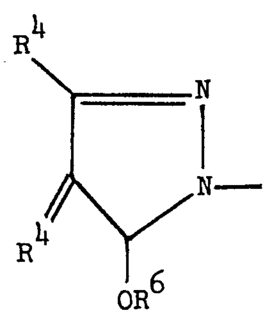

wherein

$R^3$ represents a hydrogen atom or a methyl group, each $R^4$ independently represents a hydrogen atom or a methyl, trifluoromethyl, tertiary-butyl, 2,2-dimethylpropyl or 1-methylcyclopropyl group, or, if two $R^4$ groups are present bonded to adjacent carbon atoms, the two $R^4$ groups together with the interjacent carbon atoms represent a benzene ring; each $R^5$ independently represents a hydrogen atom or a methyl group; and $R^6$ represents a 1-methylcyclopropylcarbonyl group.

7. A process for the preparation of a compound as claimed in claim 1, characterised in that one hydrogen atom of the amino group in a compound of the general formula

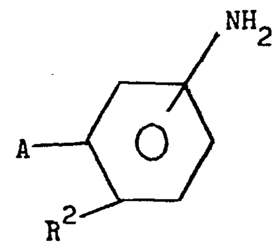

II

wherein

A and $R^2$ have the meanings given in claim 1, is replaced with a group of the general formula

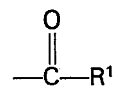

wherein

$R^1$ has the meaning given in claim 1, by reaction with a suitable acylating agent.

8. A process as claimed in claim 7, characterised in that the acylating agent is an acid chloride of the general formula

$R^1CO.Cl$

or an acid of the general formula

$R^1CO_2H$

or the anhydride thereof, wherein $R^1$ has the meaning given in claim 1.

9. A process as claimed in claim 8, characterised in that the acylating agent is an acid chloride and the reaction is carried out in the presence of an acid binding agent.

10. A herbicidal composition comprising at least one carrier, characterised in that the active ingredient is at least one compound as claimed in any one of claims 1 to 6.

11. A herbicidal composition comprising at least one carrier which is a surface-active agent, characterised in that the active ingredient is at least one compound as claimed in any one of claims 1 to 6.

12. A method of controlling undesired plant growth at a locus, characterised in that there is applied to the locus a herbicidally effective amount of a compound as claimed in any one of claims 1 to 6 or of a composition as claimed in either claim 10 or claim 11.

**Revendications**

1. Composé de la formule générale:

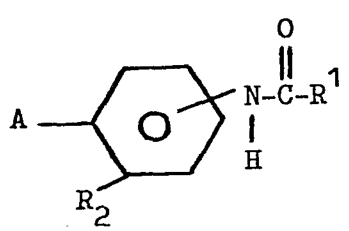

I

dans laquelle $R^1$ représente un atome d'hydrogène, un groupe cyclopropyle ayant un atome d'hydrogène, de fluor, de chlore ou de brome ou un groupe méthyle ou méthoxy dans sa position 1, ou un groupe alcoyle ayant de 1 à 4 atomes de carbone ou un groupe alcényle ayant 2 ou 3 atomes de

carbone, ces groupes pouvant être non-substitués ou substitués par un ou plusieurs atomes de fluor, de chlore et/ou de brome; $R^2$ représente un atome de fluor, de chlore ou de brome, un groupe trifluorométhyle, nitro ou cyano ou un groupe alcoyle, alcoxy ou alcoylthio ayant de 1 à 6 atomes de carbone; et A représente un des groupes:

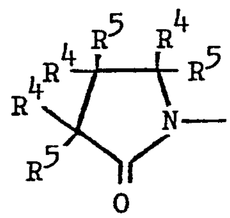
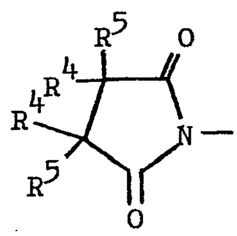
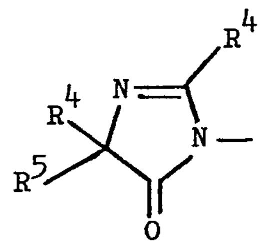
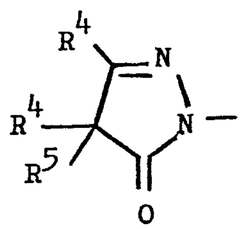
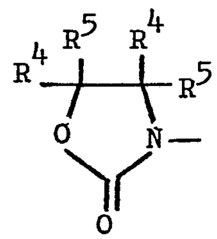
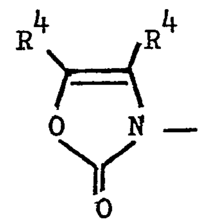
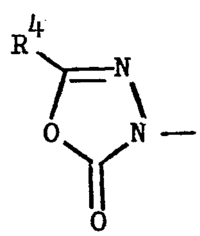
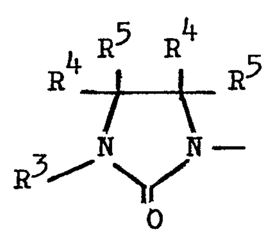
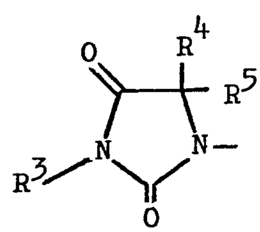
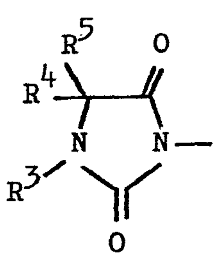
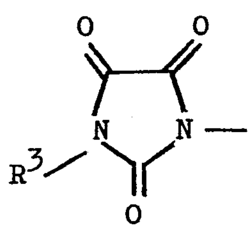
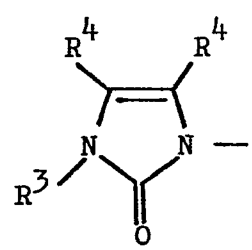
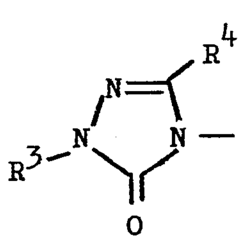
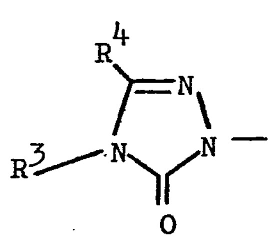
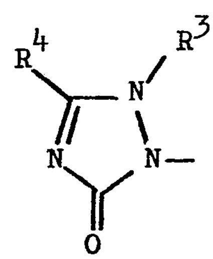

ou

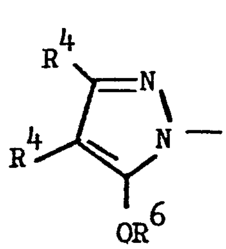

dans lesquels $R^3$ représente un atome d'hydrogène ou un groupe alcoyle ayant de 1 à 6 atomes de carbone; chaque $R^4$ indépendamment représente un atome d'hydrogène, un groupe cyclopropyle ou 1-méthylcyclopropyle ou un groupe alcoyle ayant de 1 à 6 atomes de carbone qui peut être non-substitué ou substitué par un ou plusieurs atomes d'halogènes, ou, si deux groupes $R^4$ sont présents liés à des atomes de carbone adjacents, les deux groupes $R^4$ ensemble avec les atomes de carbone interjacents représentent un noyau hydrocarbyle ayant 5 ou 6 atomes de carbone; chaque $R^5$ indépendamment représente un atome d'hydrogène ou un groupe alcoyle ayant 1 ou 2 atomes de carbone; et $R^6$ représente un groupe alcoyle ou acyle ayant de 1 à 6 atomes de carbone; ou un sel d'un tel composé.

2. Composé selon la revendication 1, caractérisé en ce que $R^1$ représente un atome d'hydrogène, un groupe alcoyle ayant de 1 à 4 atomes de carbone, un groupe alcényle ayant 2 ou 3 atomes de carbone ou un groupe cyclopropyle ayant un atome d'hydrogène, de fluor, de chlore ou de brome ou un groupe méthyle ou méthoxy dans sa position 1.

3. Composé selon la revendication 2, caractérisé en ce que le groupe:

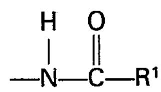

est situé dans une position méta ou para par rapport au groupe A.

4. Composé selon l'une quelconque des revendications 1 à 3, caractérisé en ce que $R^2$ représente un atome de chlore ou de brome ou un groupe méthyle ou trifluorométhyle.

5. Composé selon l'une quelconque des revendications 1 à 4, caractérisé en ce que $R^3$ représente un atome d'hydrogène ou un groupe alcoyle ayant de 1 à 4 atomes de carbone; chaque $R^4$ indépendamment représente un atome d'hydrogène, un groupe alcoyle ayant de 1 à 4 atomes de carbone ou un groupe cyclopropyle ou 1-méthylcyclopropyl, ou, si deux groupes $R^4$ sont présents liés à des atomes de carbone adjacents, les deux groupes $R^4$ ensemble avec les atomes de carbone interjacents forment un noyau hydrocarbyle ayant 5 ou 6 atomes de carbone; $R^5$ représente un atome d'hydrogène ou un groupe méthyle; et $R^6$ représente un groupe cyclopropylcarbonyle ou 1-méthylcyclopropylcarbonyle.

6. Composé selon la revendication 1, caractérisé en ce que $R^1$ représente un atome d'hydrogène ou un groupe méthyle, éthyle, tert.-butyle, 2-propen-2-yle, cyclopropyle ou 1-méthylcyclopropyle; $R^2$ représente un atome de chlore ou de brome ou un groupe trifluorométhyle et A représente un groupe de formule:

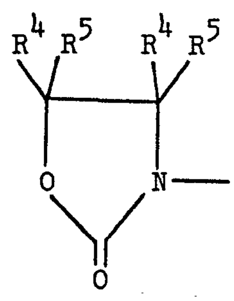
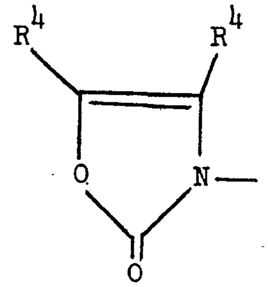
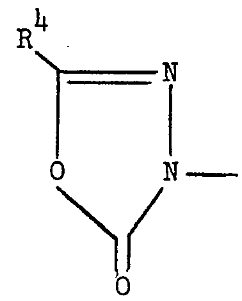
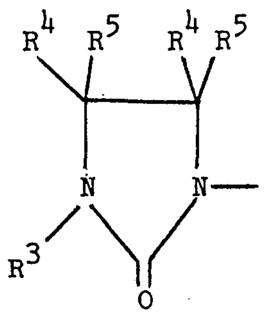
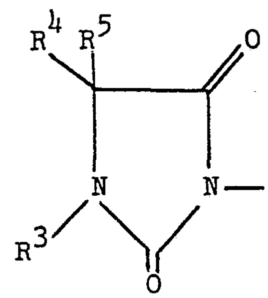
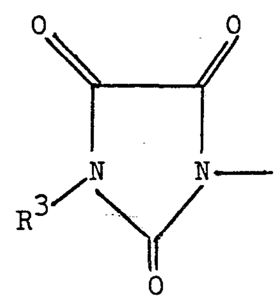
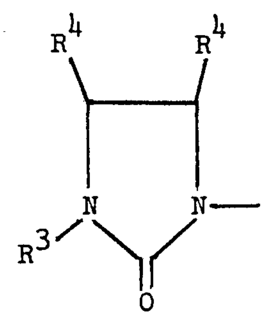

ou

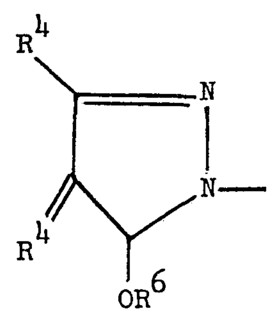

où $R^3$ représente un atome d'hydrogène ou un groupe méthyle, chaque $R^4$ indépendamment représente un atome d'hydrogène ou un groupe méthyle, trifluorométhyle, tert.-butyle, 2,2-diméthylpropyle ou 1-méthylcyclopropyle, ou, si deux groupes $R^4$ sont présents liés à des atomes de carbone adjacents, les deux groupes $R^4$ ensemble avec les atomes de carbone interjacents représentent un noyau benzénique; chaque $R^5$ indépendamment représente un atome d'hydrogène ou un groupe methyle; et $R^6$ représente un groupe 1-méthylcyclopropylcarbonyle.

7. Procédé pour la préparation d'un composé tel que revendiqué dans la revendication 1, caractérisé en ce qu'un atome d'hydrogène du groupe amino dans un composé de la formule générale:

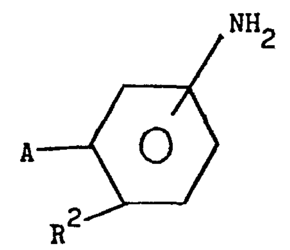

II

dans laquelle A et $R^2$ ont les significations indiquées dans la revendication 1 est remplacé par un groupe de la formule générale:

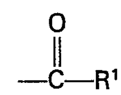

dans laquelle $R^1$ a la signification indiquée dans la revendication 1, par réaction avec un agent d'acylation approprié.

8. Procédé selon la revendication 7, caractérisé en ce que l'agent d'acylation est un chlorure d'acide de la formule générale:

$$R^1CO.Cl$$

ou un acide de la formule générale:

$$R^1CO_2H$$

ou un anhydride d'un tel acide, où $R^1$ a la signification indiquée dans la revendication 1.

9. Procédé selon la revendication 8, caractérisé en ce que l'agent d'acylation est un chlorure d'acide et que la réaction est conduite en présence d'un agent fixant les acides.

10. Composition herbicide comprenant au moins un véhicule, caractérisée en ce que l'ingrédient actif est au moins un composé tel que revendiqué dans l'une quelconque des revendications 1 à 6.

11. Composition herbicide comprenant au moins un véhicule qui est un agent tensio-actif, caractérisé en ce que l'ingrédient actif est au moins un composé tel que revendiqué dans l'une quelconque des revendications 1 à 6.

12. Procédé pour lutter contre la croissance de plantes indésirables en un lieu, caractérisé en ce qu'on applique en ce lieu une quantité efficace du point de vue herbicide d'un composé selon l'une quelconque des revendications 1 à 6 ou d'une composition selon l'une des revendications 10 et 11.

**Patentansprüche**

1. Verbindung der allgemeinen Formel:

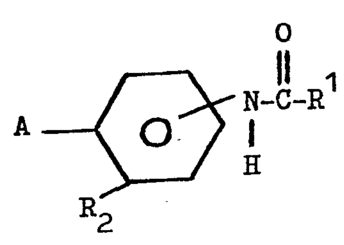

(I)

worin $R^1$ ein Wasserstoffatom, eine Cyclopropylgruppe, die in 1-Stellung ein Wasserstoff-, Fluor-, Chlor- oder Bromatom oder eine Methyl- oder Methoxygruppe trägt oder eine Alkylgruppe mit 1 bis 4 Kohlenstoffatomen oder eine Alkenylgruppe mit 2 oder 3 Kohlenstoffatomen vertritt, wobei die

erwähnten Gruppen unsubstituiert oder durch ein oder mehrere Fluor-, Chlor- und/oder Bromatome substituiert sein können; während $R^2$ ein Fluor-, Chlor- oder Bromatom, eine Trifluormethyl-, Nitro- oder Cyanogruppe oder eine Alkyl-, Alkoxy- oder Alkylthiogruppe mit 1 bis 6 Kohlenstoffatomen vertritt und A eine der folgenden Gruppen sein kann:

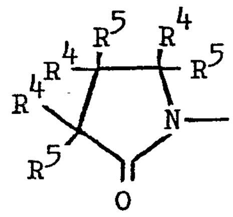
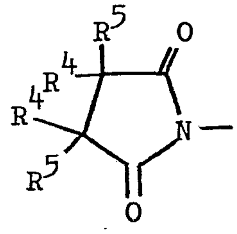
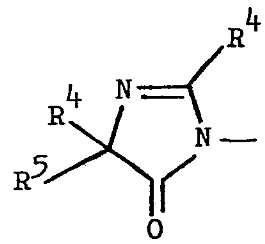
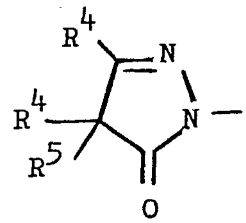
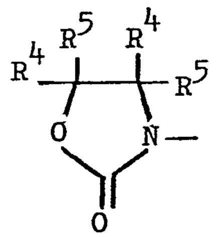
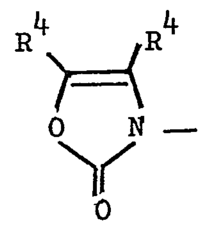
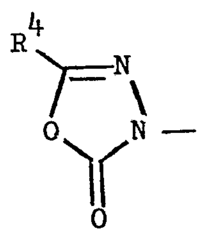
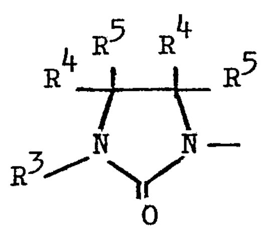
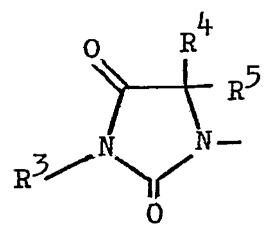
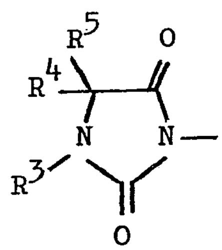
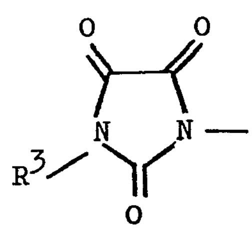
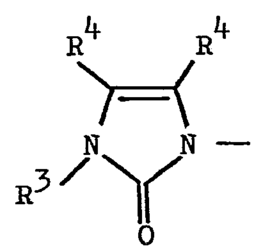
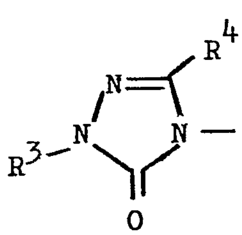
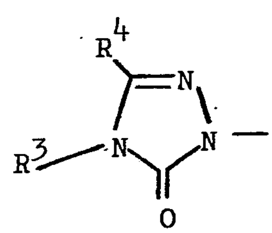
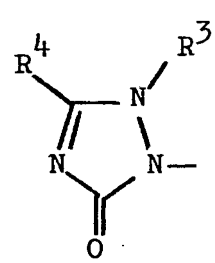

oder

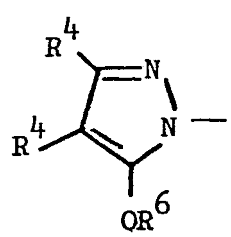

worin $R^3$ ein Wasserstoffatom oder eine Alkylgruppe mit 1 bis 6 Kohlenstoffatom darstellt und jedes $R^4$ für sich ein Wasserstoffatom oder eine Cyclopropyl- oder 1-Methyl-cyclopropylgruppe oder eine unsubstituierte oder durch ein oder mehrere Halogenatome substituierte Alkylgruppe mit 1 bis 6 Kohlenstoffatomen vertritt, oder falls zwei an benachbarte C-Atome gebundene $R^4$-Gruppen vorhanden sind, die beiden $R^4$-Gruppen gemeinsam mit den einander benachbarten C-Atomen einen Kohlenwasserstoffring mit 5 oder 6 Kohlenstoffatomen darstellen; während jedes $R^5$ für sich ein Wasserstoffatom oder eine Alkylgruppe mit 1 oder 2 Kohlenstoffatomen und $R^6$ eine Alkyl- oder Acylgruppe mit 1 bis 6 Kohlenstoffatomen vertreten, sowie Salze der obigen Verbindungen.

2. Verbindung nach Anspruch 1, dadurch gekennzeichnet, dass $R^1$ ein Wasserstoffatom, eine Alkylgruppe mit 1 bis 4 Kohlenstoffatomen, eine Alkenylgruppe mit 2 oder 3 Kohlenstoffatomen oder eine Cyclopropylgruppe mit einem Wasserstoff-, Fluor-, Chlor- oder Bromatom oder einer Methyl- oder Methoxygruppe in 1-Stellung vertritt.

3. Verbindung nach Anspruch 1 oder 2, dadurch gekennzeichnet, dass die Gruppe:

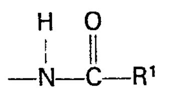

relativ zur Gruppe A die m- oder p-Stellung einnimmt.

4. Verbindung nach einem der Ansprüche 1 bis 3, dadurch gekennzeichnet, dass $R^2$ ein Chlor- oder Bromatom oder eine Methyl- oder Trifluormethylgruppe vertritt.

5. Verbindung nach einem der Ansprüche 1 bis 4, dadurch gekennzeichnet, dass $R^3$ ein Wasserstoffatom oder eine Alkylgruppe mit 1 bis 4 Kohlenstoffatomen darstellt, und jedes $R^4$ für sich ein Wasserstoffatom, eine Alkylgruppe mit 1 bis 4 Kohlenstoffatomen oder eine Cyclopropyl- oder 1-Methylcyclopropylgruppe vertritt, oder, falls zwei an benachbarte C-Atome gebundene $R^4$-Gruppen vorhanden sind, die beiden $R^4$-Gruppen gemeinsam mit den einander benachbarten C-Atomen einen Kohlenwasserstoffring mit 5 oder 6 Kohlenstoffatomen bilden; während $R^5$ ein Wasserstoffatom oder eine Methylgruppe und $R^6$ eine Cyclopropylcarbonyl- oder 1-Methylcyclopropylcarbonylgruppe bedeuten.

6. Verbindung nach Anspruch 1, dadurch gekennzeichnet, dass $R^1$ ein Wasserstoffatom oder eine Methyl-, Äthyl-, tert.-Butyl-, 2-Proplen-2-yl-, Cyclopropyl- oder 1-Methylcyclopropylgruppe vertritt, während $R^2$ ein Chlor- oder Bromatom oder eine Trifluormethylgruppe ist und A eine der folgenden Gruppen sein kann:

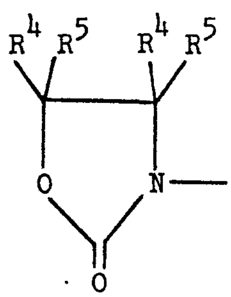

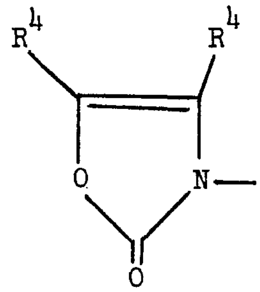

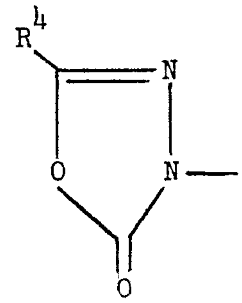

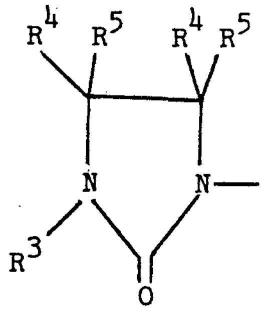

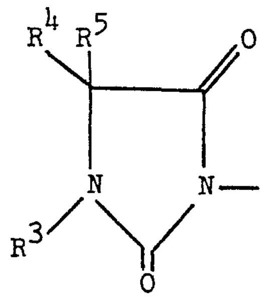

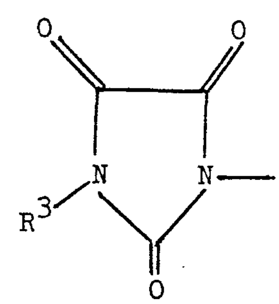

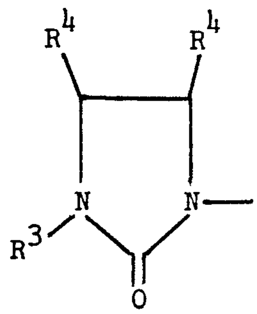

oder

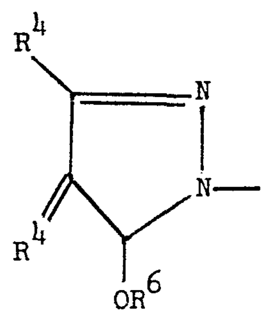

worin $R^3$ ein Wasserstoffatom oder eine Methylgruppe darstellt und jedes $R^4$ für sich ein Wasserstoffatom oder eine Methyl-, Trifluormethyl-, tert.-Butyl-, 2,2-Dimethylpropyl- oder 1-Methylcyclopropylgruppe vertritt, oder, falls zwei an benachbarte C-Atome gebundene $R^4$-Gruppen anwesend sind, diese beiden $R^4$-Gruppen gemeinsam mit den einander benachbarten C-Atomen einen Benzolring bilden; während jedes $R^5$ für sich ein Wasserstoffatom oder eine Methylgruppe und $R^6$ eine 1-Methylcyclopropylgruppe vertreten.

7. Verfahren zur Herstellung von Verbindungen nach Anspruch 1, dadurch gekennzeichnet, dass bei einer Verbindung der Formel:

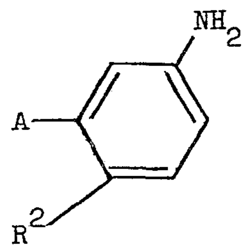

(II)

worin A und $R^2$ die in Anspruch 1 angegebene Bedeutung haben, durch Umsetzen mit einem geeigneten Acylierungsmittel ein Wasserstoffatom der Aminogruppe ersetzt wird durch eine Gruppe der allgemeinen Formel:

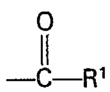

worin $R^1$ die obige Bedeutung hat.

8. Verfahren nach Anspruch 7, dadurch gekennzeichnet, dass das Acylierungsmittel ein Säurechlorid der allgemeinen Formel:

$$R^1CO.Cl$$

oder eine Säure der allgemeinen Formel:

$$R^1CO_2H$$

oder deren Anhydrid ist, wobei $R^1$ die obige Bedeutung hat.

9. Verfahren nach Anspruch 8, dadurch gekennzeichnet, dass das Acylierungsmittel ein Säurechlorid ist und dass die Reaktion in Anwesenheit eines säurebindenden Mittels durchgeführt wird.

10. Herbizides Mittel, enthaltend mindestens einen Träger, dadurch gekennzeichnet, dass der Wirkstoff mindestens eine der in einem der Ansprüche 1 bis 6 beanspruchten Verbindungen ist.

11. Herbizides Mittel, enthaltend mindestens einen Träger, der ein oberflächenaktives Mittel ist, dadurch gekennzeichnet, dass der Wirkstoff mindestens eine der in einem der Ansprüche 1 bis 6 beanspruchten Verbindungen ist.

12. Verfahren zur Bekämpfung von unerwünschtem Pflanzenwuchs an Ort und Stelle, dadurch gekennzeichnet, dass auf den Standort der Pflanzen eine der in den Ansprüchen 1 bis 6 beanspruchten Verbindungen oder eines der Mittel nach Anspruch 10 oder 11 in einer eine Herbizidwirkung entfaltenden Menge aufbringt.